# EUROPEAN PATENT SPECIFICATION

(11) **EP 3 865 591 B1**
(45) Date of publication and mention of the grant of the patent: **03.04.2024**
(21) Application number: 20157309.4
(22) Date of filing: 14.02.2020
(51) Int. Cl.: C12Q 1/6883, G01N 33/92, G01N 33/68

(54) **SET OF BIOMARKERS FOR THE DIAGNOSIS OF BRUGADA SYNDROME**
SATZ VON BIOMARKERN ZUR DIAGNOSE DES BRUGADA-SYNDROMS
ENSEMBLE DE BIOMARQUEURS POUR LE DIAGNOSTIC DU SYNDROME DE BRUGADA

(43) Date of publication of application: 18.08.2021
(73) Proprietor: Cardiomix S.r.l., 20121 Milano (IT)
(72) Inventor: PAPPONE, Carlo, 20121 Milano (IT); ANASTASIA, Luigi, 20121 Milano (IT); CICONTE, Giuseppe, 20121 Milano (IT); VICEDOMINI, Gabriele, 20121 Milano (IT)
(74) Representative: Calogero, Ida

(56) References cited:
- WO-A2-2014/152364
- GHOSH SUJOY ET AL: "Gene expression profiling in whole blood identifies distinct biological pathways associated with obesity", BMC MEDICAL GENOMICS, BIOMED CENTRAL LTD, LONDON UK, vol. 3, no. 1, 1 December 2010 (2010-12-01), page 56, XP021090959, ISSN: 1755-8794, DOI: 10.1186/1755-8794-3-56
- N. GABORIT ET AL: "Transcriptional profiling of ion channel genes in Brugada syndrome and other right ventricular arrhythmogenic diseases", EUROPEAN HEART JOURNAL, vol. 30, no. 4, 23 November 2008 (2008-11-23), pages 487-496, XP055130905, ISSN: 0195-668X, DOI: 10.1093/eurheartj/ehn520
- GOURRAUD JEAN-BAPTISTE ET AL: "Brugada syndrome: Diagnosis, risk stratification and management", ARCHIVES OF CARDIOVASCULAR DISEASE, ELSEVIER, AMSTERDAM, NL, vol. 110, no. 3, 27 January 2017 (2017-01-27), pages 188-195, XP029939358, ISSN: 1875-2136, DOI: 10.1016/J.ACVD.2016.09.009

## Description

The present invention relates to a specific set of circulating biomarkers and methods for the diagnosis of Brugada Syndrome (BrS) in a human being.

The Brugada Syndrome (BrS) is an inherited arrhythmogenic disease defined by a coved-type ST-segment elevation in the right precordial leads on the electrocardiogram and increased risk of sudden cardiac death (SCD) in patients with structurally normal hearts [1-4].

Brugada Syndrome has been reported to be responsible for 5-40% of sudden cardiac deaths and is an important cause of death in individuals aged <40 years [5-7], even if it occurs also in infants and children [1]. The syndrome is endemic in Asiatic regions [6] and appears to be 8 to 10 times more common in men than women.

The syndrome typically manifests with cardiac arrest or syncope, occurring in the third and fourth decade of life [3, 4, 6], however, the majority of patients are asymptomatic with structurally normal heart, and they are usually diagnosed incidentally.

The risk stratification of Brugada Syndrome, particularly in asymptomatic patients, still represents a clinical challenge.

It was demonstrated that the extent of the arrhythmic substrate is the only independent predictor of inducibility of ventricular fibrillation (VF) and ventricular tachycardia (VT) and might be considered as a new marker for risk stratification and therapy [8].

Currently, subjects with spontaneous type 1 ECG pattern and aborted sudden cardiac death or syncope of arrhythmic origin are at highest risk for future arrhythmic events and are advised to receive an implantable cardioverter defibrillator (ICD), despite the risk of device-related complications and inappropriate shocks.

To date Brugada Syndrome diagnosis is based on the identification of the typical ECG-trace signal that may be spontaneous (type 1) or can be induced by a pharmacological challenge test with intravenous administration of sodium channel blockers, such as ajmaline or flecainide. To be considered as diagnostic for BrS, the pattern seen on the ECG includes coved-type ST elevation in the right precordial leads, mainly V1 and V2 placed in the high right intercostal spaces (from the second to the fourth).

The ajmaline challenge is the most accurate test to-date to diagnose Brugada Syndrome in individuals without a spontaneous type 1 ECG pattern. In patients with normal cardiac cells, ajmaline has little or no effect on the ECG. The drug has proven to be a powerful tool in unmasking the Brugada type 1 pattern [10] and to be more accurate than flecainide, which is associated with a 36% false negative rate [11]. However, ajmaline administration can provoke VT/VF during the diagnostic procedure. As a result, the Brugada diagnostic test with ajmaline challenge may be also not allowed in several countries as screening testfor safety reasons. Moreover, given the risk associated with the procedure, patients themselves refuse to undergo the procedure. Additionally, the procedure requires an experienced staff, in hospitals with an Extra Corporeal Membrane Oxygenation (ECMO) team. Therefore, this procedure is not widely available, implying that patients may need to travel long distances for the procedure. From an economical perspective, the procedure is expensive and a considerable burden for the Italian National Healthcare System. As a result, potential patients do not undergo the diagnostic test in a timely fashion, or at all, exposing them to considerable risk of sudden cardiac death.

Moreover, Brugada Syndrome has a further not negligible genetic treat: it is a heterogeneous channelopathy, inherited as an autosomal dominant trait with incomplete penetrance [9].

To date, about three hundred pathogenic variants in 25 genes have been identified in subjects with Brugada Syndrome, but most of them (25-30%) involve the *SCN5A* gene on chromosome 3p22, encoding the a-subunit of the cardiac sodium channel NaV1.5 [10]. Other genetic lesions associated with Brugada Syndrome include heterozygous variants in *CACNA1C, SCN10A, PKP2, TRPM4, KCNH2,* and at least eighteen other genes. *SCN5A* heterozygous mutations account for 11-28% of all Brugada Syndrome cases [11].

The international patent application WO2014/152364 A1 discloses a method of identifying the presence of Brugada Syndrome in a subject by detecting (a) the level of full-length mRNA encoded by the SCN5A gene, (b) the level of mRNA of an SCN5A splice variant which encodes a truncated SCN5A protein, (c) the level of mRNA encoded by a SCN5A splicing factor gene, and/or (d) a level of mRNA encoded by a gene of the unfolded protein response (UPR); and identifying the presence of the syndrome when the level of (a), (b), and/or (d) are reduced, relative to a control level, and/or when the level of (c) is increased, relative to a control level.

However, about 90% of patients with Brugada Syndrome do not have a mutation in the SCN5A gene.

To further complicate the genetic assessment, there are different related Brugada Syndromes caused by mutations in genes other than the SCN5A gene. For example, Brugada Syndrome-2 (Phenotype MIM #611777) is caused by mutation in the GPD1L gene. Brugada Syndrome-3 (Phenotype MIM #611875) and Brugada Syndrome-4 (Phenotype MIM #611876), the phenotypes of which include a shortened QT interval on ECG, are caused by mutation in the CACNA1C and CACNB2 genes, respectively. Brugada Syndrome-5 (Phenotype MIM #612838) is caused by mutation in the SCN1B gene. Brugada Syndrome-6 (Phenotype MIM #613119) is caused by mutation in the KCNE3 gene. Brugada Syndrome-7 (Phenotypte MIM #613120) is caused by mutation in the SCN3B gene. Brugada syndrome-8 (Phenotype MIM #613123) is caused by mutation in the HCN4 gene. While the scientific community still regards Brugada Syndrome as an ion channelopathy, further evidence suggests that the syndrome may also originate by additional factors. For example, an alpha-tropomyosin heterozygous mutation appears to link HCM and Brugada Syndrome in one study, suggesting a role for sarcomeropathies [12]. Another study, published by the present inventors, demonstrated for the first time a pathogenic variant in the *MYBPC3* gene and its potential association with Brugada Syndrome [13]. In fact, altered sarcomeric properties have been directly implicated in arrhythmogenesis and sudden death [14-16].

In the light of the complexity of this multi-factorial disorder and the existence of many genetic variants of the disease at the molecular level, genetic testing may be used just to confirm a clinical diagnosis of Brugada Syndrome but is not suitable to make diagnosis of the disease in any subject.

These limitations of genetic screening highlight the urgent need to seek biomarkers aimed both at confirmation of the Brugada Syndrome to establish a prognosis in already affected patients.

In this direction, the systematic introduction of active screening in unmasking Brugada Syndrome using sodium-channel blockers, has considerably increased the diagnosis and incidence of the disease, although its true prevalence is still unknown [17]. Nonetheless, the drug challenge used for the diagnosis can be associated with the occurrence of potentially life-threatening ventricular arrhythmias, thus limiting its widespread use in the absence of a safe hospital environment.

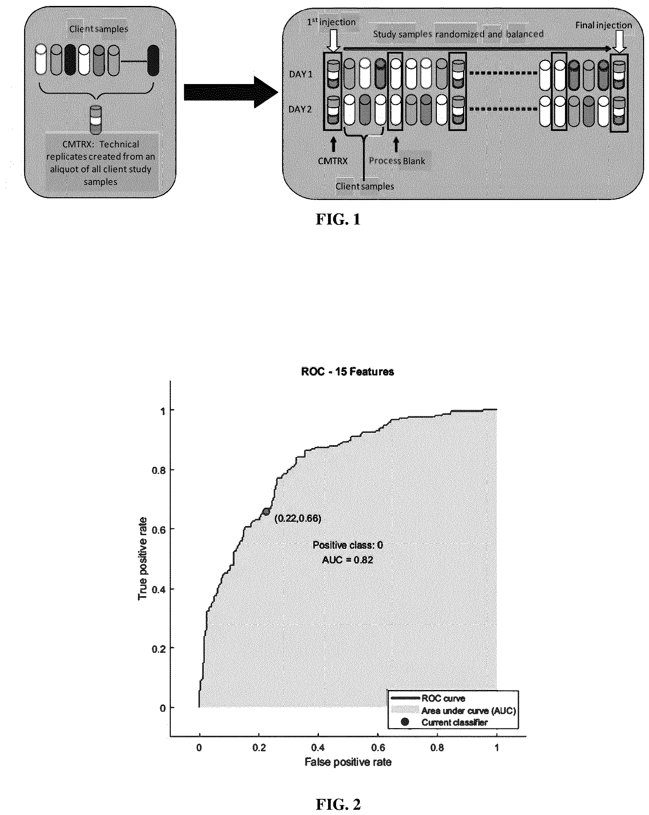

Gaborit N. et al., 2008 discloses gene expression signatures diagnostic for Brugada syndrome (see Figures 1 and 2) providing for an overall accuracy of 72% with a specificity of 65.7% and a sensitivity of 77.6%.

In view of the foregoing, there is a need for a more time and cost-efficient, sensitive and non-invasive laboratory test for diagnosing Brugada Syndrome, which could eventually replace the drug challenge, or at least limit it to those individuals that are positive to such initial laboratory screening and overcome the limits of genetic screening.

The authors of the present invention have now identified a set of circulating biomarkers that allows to overcome the different technical problems affecting the prior art, providing a reliable and sensitive method for the diagnosis of Brugada Syndrome in any patients, herein included also asymptomatic subjects. Specifically, the authors found that patients affected by Brugada Syndrome possess signs of the disease that are detectable in the peripheral blood. In particular, based on the increasing number and diversity of the mutated genes found in patients affected by Brugada Syndrome, the authors hypothesized that the disease could affect cell compartments other than those present in the heart. To this end, considering the complexity of the pathology and the heterogeneity among the patient population, they applied a multi-omics (genomic, transcriptomic, and metabolomics) characterization of peripheral plasma and PBMCs on a large scale, supported by an extensive and innovative bioinformatics and computational platforms, leading to the identification of a univocal set of biomarkers, statistically significant related to Brugada Syndrome.

A significant advantage of the use of the set of biomarkers of the invention includes the diagnostic and prognostic utility in a wide set of subtypes of Brugada Syndrome, regardless of the genotype or expression of other known genetic biomarker, such as the SCN5A gene.

The set of biomarkers of the invention may also be very useful for risk certification of the patients with Brugada syndrome. Furthermore, a positive result with the set of biomarkers of the invention allows to identify patients affected by the Brugada disease, independently from being symptomatic or not.

Therefore, it is an object of the present invention a set of 14 biomarkers comprising at least:
i) the subset of 6 mRNA listed in the following Table 1:

**TABLE 1**

| **Name** | **Ensemble ID** |
|---|---|
| Calpain 2 (CAPN2) | ENSG00000162909 |
| Dynactin Subunit 2 (DCTN2) | ENSG00000175203 |
| Diacylglycerol Kinase Delta (DGKD) | ENSG00000077044 |
| Major Histocompatibility Complex, Class II, DP Alpha 1 (HLA-DPA1) | ENSG00000231389 |
| Major Histocompatibility Complex, Class II, DP Beta 1 (HLA-DPB1) | ENSG00000223865 |
| WD Repeat Domain 86 (WDR86) | ENSG00000187260 |

ii) the subset of the following 7 metabolites:
- 2-aminooctanoate
- 4-methoxyphenol sulfate
- hypotaurine
- lysine
- N-acetyl-2-aminooctanoate
- phenol sulfate
- γ-glutamylleucine;
   and

iii) the lipid dodecanedioate (C12:1-DC); for the diagnosis of Brugada syndrome in a human being.

The set of 14 biomarkers according to the present invention allows a diagnosis of the disease of Brugada syndrome on PBMC population of any subject with an overall accuracy of about 72-75%.

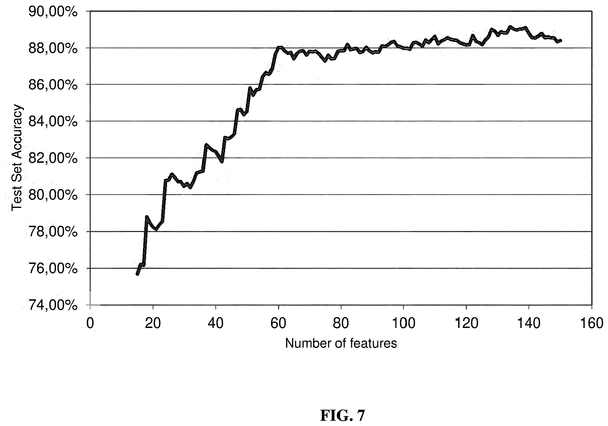

By increasing the number of biomarkers from 14 to 59 it is possible to reach an overall accuracy of the test on PBMC population of about 87%. When more than 60 biomarkers are used a plateau in the accuracy of the test is observed (see Figure 7).

The subset of the invention may further comprise additional biomarkers belonging to the above listed subsets i)-iii), not exceeding the number of 59.

Therefore, it is another object of the present invention a set of 14 biomarkers further comprising one or more of the following additional biomarkers:
i) RNA selected from the group consisting of:

**TABLE 2**

| **Name** | **Ensemble ID** | **Type** |
|---|---|---|
| Acyl-CoA Synthetase Family Member 4 (AASDH) | ENSG00000157426 | mRNA |
| Ankyrin Repeat And Death Domain Containing 1A (ANKDD1A) | ENSG00000166839 | mRNA |
| Ankyrin Repeat Domain 49 (ANKRD49) | ENSG00000168876 | mRNA |
| Rho/Rac Guanine Nucleotide Exchange Factor 18 (ARHGEF18) | ENSG00000104880 | mRNA |
| BRICHOS Domain Containing 5 (BRICD5) | ENSG00000182685 | mRNA |
| BRCA1 Interacting Protein C-Terminal Helicase 1 (BRIP1) | ENSG00000136492 | mRNA |
| BRO1 Domain And CAAX Motif Containing (BROX) | ENSG00000162819 | mRNA |
| Glucocorticoid Receptor AF-1 Coactivator-1 (BTBD6) | ENSG00000184887 | mRNA |
| Chromosome 19 Open Reading Frame 79 (C12orf79) | ENSG00000257242 | Long noncoding RNA |
| Chromosome 19 Open Reading Frame 38 (C19orf38) | ENSG00000214212 | mRNA |
| Calcium/Calmodulin Dependent Protein Kinase Kinase 2 (CAMKK2) | ENSG00000110931 | mRNA |
| Chimerin I (CHN1) | ENSG00000128656 | mRNA |
| Cyclin-M3 (CNNM3) | ENSG00000168763 | mRNA |
| Cytochrome P450 Family 2 Subfamily S Member 1 (CYP2S1) | ENSG00000167600 | mRNA |
| ERBB Receptor Feedback Inhibitor 1 (ERRFI1) | ENSG00000116285 | mRNA |
| Retrotransposon Gag Like 8C (FAM 127A) | ENSG00000134590 | mRNA |
| Glycoprotein, Alpha-Galactosyltransferase (GGTA1P) | ENSG00000204136 | mRNA |
| Glutathione-Disulfide | ENSG00000104687 | mRNA |
| Reductase (GSR) | | |
| HLA Complex Group 18 (HCG18) | ENSG00000231074 | mRNA |
| Histone Cluster 1 H1 Family Member C (HIST1H1C) | ENSG00000187837 | mRNA |
| Histone Cluster 1 H2B Family Member K (HIST1H2BK) | ENSG00000197903 | mRNA |
| Insulin Like Growth Factor Binding Protein 3 (IGFBP3) | ENSG00000146674 | mRNA |
| Ras GTPase-Activating-Like Protein (IQGAP2) | ENSG00000145703 | mRNA |
| Lymphotoxin Beta (LTB) | ENSG00000227507 | mRNA |
| Methionine Adenosyltransferase 2A (MAT2A) | ENSG00000168906 | mRNA |
| Mitochondrial Ribosomal Protein L22 (MRPS22) | ENSG00000175110 | mRNA |
| Nucleosome Assembly Protein 1 Like 2 (NAP1L2) | ENSG00000186462 | mRNA |
| Peptidyl Arginine Deiminase 4 (PADI4) | ENSG00000159339 | mRNA |
| PDZ And LIM Domain 7 (PDLIM7) | ENSG00000196923 | mRNA |
| Platelet Activating Factor Receptor (PTAFR) | ENSG00000169403 | mRNA |
| RAN Binding Protein 6 (RANBP6) | ENSG00000137040 | mRNA |
| RNA, 7SL, Cytoplasmic 473, Pseudogene (RN7SL473P) | ENSG00000277452 | mRNA |
| RP11-429E11.3 | ENSG00000179253.3 | mRNA |
| RP11-4B16.1 | ENSG00000279433 | microRNA |
| Ribosomal Protein S20 (RPS20) | ENSG00000008988 | mRNA |
| Syntaxin 10 (STX10) | ENSG00000104915 | mRNA |
| T Cell Receptor Beta Variable 11-2 (TRBV11-2) | ENSG00000241657 | mRNA |
| Zwilch Kinetochore Protein (ZWILCH) | ENSG00000174442 | mRNA |

ii) metabolite selected from the group consisting of:
- 5-methyluridine (ribothymidine)
- fibrinopeptide A, phosphono-ser(3)
- glyco-alpha-muricholate;
- 3-(4-hydroxyphenyl)lactate
- glucoronate
- Trans-4-hydroxyproline;

iii) phosphatidylinositol (16:0/18:1); or a combination thereof.

Preferably, all the additional biomarkers above listed are included in the set of biomarkers according to the invention, albeit also intermediate solutions wherein only some of them are included are contemplated in the scope of the present invention.

Therefore, according to a further preferred embodiment it is another object of the present invention a set of 59 biomarkers comprising:
i) the subset of 44 RNA listed in the following Table 3:

**TABLE 3**

| **Name** | **Ensemble ID** | **Type** |
|---|---|---|
| Acyl-CoA Synthetase Family Member 4 (AASDH) | ENSG00000157426 | mRNA |
| Ankyrin Repeat And Death Domain Containing 1A (ANKDD1A) | ENSG00000166839 | mRNA |
| Ankyrin Repeat Domain 49 (ANKRD49) | ENSG00000168876 | mRNA |
| Rho/Rac Guanine Nucleotide Exchange Factor 18 (ARHGEF18) | ENSG00000104880 | mRNA |
| BRICHOS Domain Containing 5 (BRICD5) | ENSG00000182685 | mRNA |
| BRCA1 Interacting Protein C-Terminal Helicase 1 (BRIP1) | ENSG00000136492 | mRNA |
| BRO1 Domain And CAAX Motif Containing (BROX) | ENSG00000162819 | mRNA |
| Glucocorticoid Receptor AF-1 Coactivator-1 (BTBD6) | ENSG00000184887 | mRNA |
| Chromosome 19 Open Reading Frame 79 (C12orf79) | ENSG00000257242 | Long noncoding RNA |
| Chromosome 19 Open Reading Frame 38 (C19orf38) | ENSG00000214212 | mRNA |
| Calcium/Calmodulin Dependent Protein Kinase Kinase 2 (CAMKK2) | ENSG00000110931 | mRNA |
| Calpain 2 (CAPN2) | ENSG00000162909 | mRNA |
| Chimerin I (CHN1) | ENSG00000128656 | mRNA |
| Cyclin-M3 (CNNM3) | ENSG00000168763 | mRNA |
| Cytochrome P450 Family 2 Subfamily S Member 1 (CYP2S1) | ENSG00000167600 | mRNA |
| Dynactin Subunit 2 (DCTN2) | ENSG00000175203 | mRNA |
| Diacylglycerol Kinase Delta (DGKD) | ENSG00000077044 | mRNA |
| ERBB Receptor Feedback Inhibitor 1 (ERRFI1) | ENSG00000116285 | mRNA |
| Retrotransposon Gag Like 8C (FAM 127A) | ENSG00000134590 | mRNA |
| Glycoprotein, Alpha-Galactosyltransferase (GGTA1P) | ENSG00000204136 | mRNA |
| Glutathione-Disulfide Reductase (GSR) | ENSG00000104687 | mRNA |
| HLA Complex Group 18 (HCG18) | ENSG00000231074 | mRNA |
| Histone Cluster 1 H1 Family Member C (HIST1H1C) | ENSG00000187837 | mRNA |
| Histone Cluster 1 H2B Family Member K (HIST1H2BK) | ENSG00000197903 | mRNA |
| Major Histocompatibility Complex, Class II, DP Alpha 1 (HLA-DPA1) | ENSG00000231389 | mRNA |
| Major Histocompatibility Complex, Class II, DP Beta 1 (HLA-DPB1) | ENSG00000223865 | mRNA |
| Insulin Like Growth Factor Binding Protein 3 (IGFBP3) | ENSG00000146674 | mRNA |
| Ras GTPase-Activating-Like Protein (IQGAP2) | ENSG00000145703 | mRNA |
| Lymphotoxin Beta (LTB) | ENSG00000227507 | mRNA |
| Methionine Adenosyltransferase 2A (MAT2A) | ENSG00000168906 | mRNA |
| Mitochondrial Ribosomal Protein L22 (MRPS22) | ENSG00000175110 | mRNA |
| Nucleosome Assembly Protein 1 Like 2 (NAP1L2) | ENSG00000186462 | mRNA |
| Peptidyl Arginine Deiminase 4 (PADI4) | ENSG00000159339 | mRNA |
| PDZ And LIM Domain 7 (PDLIM7) | ENSG00000196923 | mRNA |
| Platelet Activating Factor Receptor (PTAFR) | ENSG00000169403 | mRNA |
| RAN Binding Protein 6 (RANBP6) | ENSG00000137040 | mRNA |
| RNA, 7SL, Cytoplasmic 473, Pseudogene (RN7SL473P) | ENSG00000277452 | mRNA |
| RP11-429E11.3 | ENSG00000179253.3 | mRNA |
| RP11-4B16.1 | ENSG00000279433 | microRNA |
| Ribosomal Protein S20 (RPS20) | ENSG00000008988 | mRNA |
| Syntaxin 10 (STX10) | ENSG00000104915 | mRNA |
| T Cell Receptor Beta Variable 11-2 (TRBV11-2) | ENSG00000241657 | mRNA |
| WD Repeat Domain 86 (WDR86) | ENSG00000187260 | mRNA |
| Zwilch Kinetochore Protein (ZWILCH) | ENSG00000174442 | mRNA |

ii) the subset of the following 13-metabolites:
- 2-aminooctanoate
- 3-(4-hydroxyphenyl)lactate
- 4-methoxyphenol sulfate
- hypotaurine
- lysine
- glucoronate
- Trans-4-hydroxyproline
- N-acetyl-2-aminooctanoate
- phenol sulfate
- γ-glutamylleucine
- 5-methyluridine (ribothymidine)
- fibrinopeptide A, phosphono-ser(3)
- glyco-alpha-muricholate;

and iii) the subset of the following lipids:
   - dodecanedioate (C12:1-DC)
   - phosphatidylinositol (16:0/18:1);
for the diagnosis of Brugada Syndrome in a human being.

It is another object of the present invention an in vitro method for detecting the presence or measuring concentration of the occurrence of one of the set of biomarkers above listed in a biological sample of a human being.

In order to provide a reliable result, the in vitro method should at least foresee the detection of the presence or measuring concentration of at least the 14 set of biomarkers up to the 59 set of biomarkers.

In a particular embodiment the invention provides an in vitro method for detecting the presence of one of the set of biomarkers above listed in a biological sample of a human being, comprising the following steps:
a) detection of at least 6 up to 44 RNAs of subset i) by an assay selected from the group comprising mRNA microarray detection, Real-Time PCR, Droplet Digital PCR or hybridization-based assays, and branched DNA (bDNA) technology (i.e. QuantiGene^{®} assay), or a combination thereof; and
b) detection of at least 7 up to 13 of the metabolites of subset ii) and of one or both the lipids of subset iii) by an assay such as one selected from the group comprising MS, GC-MS, fluorimetric or colorimetric assays, NMR, spectroscopy with nanoprobes, Enzyme-Linked oligonucleotide assays, or a combination thereof; wherein the positive detection of all the biomarkers compared to a normal control allows the identification of patients affected by Brugada Syndrome.

According to an alternative embodiment the invention relates to an in vitro method for measuring the concentration of one of the above listed set of biomarkers in a biological sample of a human being, comprising the following steps:
a) quantitative determination of at least 6 up to 44 RNAs of subset i) by a quantitative technique such as quantitative PCR, preferably by Real Time-PCR, Droplet Digital PCR, Quantigene assay, or a combination thereof; and
b) quantitative determination of at least 7 up to 13 of the metabolites of subset ii) by a technique such as HPLC, MS, NMR analysis, ELONA, or a combination thereof;
c) quantitative determination of one or both the lipids of subset iii) by a technique such as HPLC, MS, NMR analysis, or a combination thereof;
wherein an increase of the concentration of the biomarkers compared to a normal control indicates a diagnosis of Brugada Syndrome.

A positive result to all biomarkers means that the patient is affected by Brugada Syndrome, independently on the fact that such patient is asymptomic or symptomatic.

According to a preferred embodiment of the invention said biological sample is selected from the group consisting of whole blood, plasma, serum, peripheral blood and PBMCs. Preferably, the biological sample is plasma and/or PBMCs. According to alternative embodiments of the aforementioned method, the starting biological sample can come indifferently from adult subjects or children, male or female subjects.

In a preferred embodiment of the invention said human being may be either asymptomatic (that is with structurally normal heart) or at high risk for Brugada Syndrome due to family history, previous events of heart atrial and/or ventricular fibrillation, diabetes or obesity.

More preferably said human being is about 40 years old or younger.

The invention is further directed to a kit for the detection of the subset i) of at least 6 mRNA of Table 1 up to the subset i) of 44 RNAs of Table 3, said kit comprising oligonucleotides that are at least 85, 90, 95, 96, 97, 98, 99 or 100% complementary to each of the mRNA sequences i), wherein said oligonucleotides are primers or probes optionally attached to a solid support. Also the intermediate combination with one or more of the 38 RNAs listed in Table 2 are contemplated in this embodiment of the invention.

The present invention will now be described, for non-limiting illustrative purposes, according to a preferred embodiment thereof, with particular reference to the attached figures, wherein:
- Figure 1 shows a scheme of the procedure to create a CMTRX technical replicate sample.
- Figure 2 shows the ROC curve of the multiomic Brugada Syndrome set of 15 features (14 biomarkers + patient age) of the invention.
- Figure 3 shows the specificity and sensitivity of the Brugada Syndrome multi-omics 15-feature panels (metabolomics, lipidomics, transcriptomics) of the invention.
- Figure 4 shows the ROC curve of the multiomic Brugada Syndrome set of 60 features of the invention (59 biomarkers+ patient age).
- Figure 5 shows the specificity and sensitivity of the Brugada Syndrome multi-omics 60 features panels (metabolomics, lipidomics, transcriptomics) of the invention.
- Figure 6 shows the curve of the accuracy of the test carried out with less than 60 features.
- Figure 7 shows the plateau reached by the curve of the accuracy of the test when more than 60 features have been employed.

The following example is merely illustrative and should not be considered limiting the scope of the present invention.

### EXAMPLE 1: Multi-omic identification of the set of biomarkers of Brugada syndrome of the invention

### MATERIALS AND METHODS

### Ethical Statement

All procedures performed in studies involving human participants were in accordance with the ethical standards of the institutional and/or national research committee and with the Helsinki declaration and its later amendments or comparable ethical standards. The study design was examined by the Ethical Committee of the San Raffaele Hospital (Protocol BASED-version 07/01/2004).

### Patients enrollment

The population study has been constituted by two groups (300 subjects/group): the Brugada (BrG) and the Control (CG) Groups. The expected enrollment period was of 24 months.

Patients were evaluated for:
- baseline laboratory examinations, including total blood count, hepatic, thyroid and coagulation function.
- transthoracic ECG.

The inclusion/exclusion criteria for the two groups have been respectively the following.

Brugada Group: All consecutive Brugada Syndrome patients, aged >18 years, positive to the ajmaline test, or considered at high risk of SCD and undergoing epicardial ablation will be enrolled in this study.

Control Group: A population of patients with a structurally normal heart with a negative ajmaline test confirming the absence of Brugada Syndrome.

Inclusion criteria for Control Group:
- Age >18 years
- Absence of Brugada Syndrome confirmed with ajmaline test.

Exclusion criteria for Control Group:
- Ejection Fraction <55%
- Coronary artery disease with the need for revascularization
- Previous heart surgery
- Recent surgical/percutaneous intervention for valvular heart disease (<6 months)
- Life expectancy <1 year
- Impossibility to provide consent to the procedure and to the study

### Transcriptomic analysis

Transcriptomic analysis was performed at the Center for Translational Genomics and Bioinformatics at San Raffaele Hospital.

RNA quality was confirmed with a 2100 Bioanalyzer (Agilent) and RNA with a RIN above 7 were included in the analysis. To generate the libraries, the TruSeq stranded mRNA protocol was performed. This protocol allowed unbiased 5'/3' library preparation starting from 100 ng of total RNA. Libraries were barcoded, pooled and sequenced on an Illumina Nova-Seq 6000 sequencer. RNA-Seq experiments were performed generating single-end 30M reads, 75nt long, for each run. After trimming of adapters, sequences generated within RNA-Seq experiments were aligned to the genome using the STAR aligner (20) and counted with feature Counts (21) on the appropriate annotation: genes from the last Gencode (22) release for RNA-Seq.

### Metabolomic analysis on plasma

Samples were prepared using the automated MicroLab STAR^{®} system from Hamilton Company. Several recovery standards were added prior to the first step in the extraction process for QC purposes. To remove protein, dissociate small molecules bound to protein or trapped in the precipitated protein matrix, and to recover chemically diverse metabolites, proteins were precipitated with methanol under vigorous shaking for 2 min (Glen Mills GenoGrinder 2000) followed by centrifugation. The resulting extract was divided into five fractions: two for analysis by two separate reverse phase (RP)/UPLC-MS/MS methods with positive ion mode electrospray ionization (ESI), one for analysis by RP/UPLC-MS/MS with negative ion mode ESI, one for analysis by HILIC/UPLC-MS/MS with negative ion mode ESI, and one sample was reserved for backup. Samples were placed briefly on a TurboVap^{®} (Zymark) to remove the organic solvent. The sample extracts were stored overnight under nitrogen before preparation for analysis.

### QA/QC

Several types of controls were analyzed in concert with the experimental samples: a pooled matrix sample generated by taking a small volume of each experimental sample (or alternatively, use of a pool of well-characterized human plasma) served as a technical replicate throughout the data set; extracted water samples served as process blanks; and a cocktail of QC standards that were carefully chosen not to interfere with the measurement of endogenous compounds were spiked into every analyzed sample, allowed instrument performance monitoring and aided chromatographic alignment.

The following Tables 4 and 5 describe these QC samples and standards.

**TABLE 4: Description of Metabolon QC Samples**

| Type | Description | Purpose |
|---|---|---|
| MTRX | Large pool of human plasma maintained by Metabolon that has been characterized extensively. | Assure that all aspects of the Metabolon process are operating within specifications. |
| CMTRX | Pool created by taking a small aliquot from every customer sample. | Assess the effect of a non-plasma matrix on the Metabolon process and distinguish biological variability from process variability. |
| PRCS | Aliquot of ultra-pure water | Process Blank used to assess the contribution to compound signals from the process. |
| SOLV | Aliquot of solvents used in extraction. | Solvent Blank used to segregate contamination sources in the extraction. |

**TABLE 5: Metabolon QC Standards**

| Type | Description | Purpose |
|---|---|---|
| RS | Recovery Standard | Assess variability and verify performance of extraction and instrumentation. |
| IS | Internal Standard | Assess variability and performance of instrument. |

Instrument variability was determined by calculating the median relative standard deviation (RSD) for the standards that were added to each sample prior to injection into the mass spectrometers. Overall process variability was determined by calculating the median RSD for all endogenous metabolites (i.e., non-instrument standards) present in 100% of the pooled matrix samples. Experimental samples were randomized across the platform run with QC samples spaced evenly among the injections, as outlined in Figure 1. Figure 1 shows the preparation of client-specific technical replicates wherein a small aliquot of each client sample (colored cylinders) is pooled to create a CMTRX technical replicate sample (multi-colored cylinder), which is then injected periodically throughout the platform run. Variability among consistently detected biochemicals can be used to calculate an estimate of overall process and platform variability.

### Ultrahigh Performance Liquid Chromatography-Tandem Mass Spectroscopy (UPLC-MS/MS)

All methods utilized a Waters ACQUITY ultra-performance liquid chromatography (UPLC) and a Thermo Scientific Q-Exactive high resolution/accurate mass spectrometer interfaced with a heated electrospray ionization (HESI-II) source and Orbitrap mass analyzer operated at 35,000 mass resolution. The sample extract was dried then reconstituted in solvents compatible to each of the four methods. Each reconstitution solvent contained a series of standards at fixed concentrations to ensure injection and chromatographic consistency. One aliquot was analyzed using acidic positive ion conditions, chromatographically optimized for more hydrophilic compounds. In this method, the extract was gradient eluted from a C18 column (Waters UPLC BEH C18-2.1x100 mm, 1.7 µm) using water and methanol, containing 0.05% perfluoropentanoic acid (PFPA) and 0.1% formic acid (FA). Another aliquot was also analyzed using acidic positive ion conditions, however it was chromatographically optimized for more hydrophobic compounds. In this method, the extract was gradient eluted from the same afore mentioned C18 column using methanol, acetonitrile, water, 0.05% PFPA and 0.01% FA and was operated at an overall higher organic content. Another aliquot was analyzed using basic negative ion optimized conditions using a separate dedicated C18 column. The basic extracts were gradient eluted from the column using methanol and water, however with 6.5mM Ammonium Bicarbonate at pH 8. The fourth aliquot was analyzed via negative ionization following elution from a HILIC column (Waters UPLC BEH Amide 2.1x150 mm, 1.7 µm) using a gradient consisting of water and acetonitrile with 10mM Ammonium Formate, pH 10.8. The MS analysis alternated between MS and data-dependent MSn scans using dynamic exclusion. The scan range varied slighted between methods but covered 70-1000 m/z. Raw data files are archived and extracted as described below.

### Data Extraction and Compound Identification

Raw data was extracted, peak-identified and QC processed using Metabolon's hardware and software. Compounds were identified by comparison to library entries of purified standards or recurrent unknown entities. Metabolon maintains a library based on authenticated standards that contains the retention time/index (RI), mass to charge ratio (m/z), and chromatographic data (including MS/MS spectral data) on all molecules present in the library. Furthermore, biochemical identifications are based on three criteria: retention index within a narrow RI window of the proposed identification, accurate mass match to the library +/- 10 ppm, and the MS/MS forward and reverse scores between the experimental data and authentic standards. The MS/MS scores are based on a comparison of the ions present in the experimental spectrum to the ions present in the library spectrum. While there may be similarities between these molecules based on one of these factors, the use of all three data points can be utilized to distinguish and differentiate biochemicals. More than 3300 commercially available purified standard compounds have been acquired and registered for analysis on all platforms for determination of their analytical characteristics. Additional mass spectral entries have been created for structurally unnamed biochemicals, which have been identified by virtue of their recurrent nature (both chromatographic and mass spectral). These compounds have the potential to be identified by future acquisition of a matching purified standard or by classical structural analysis.

### Curation

A variety of curation procedures were carried out to ensure that a high quality data set was made available for statistical analysis and data interpretation. The QC and curation processes were designed to ensure accurate and consistent identification of true chemical entities, and to remove those representing system artifacts, mis-assignments, and background noise. Library matches for each compound were checked for each sample and corrected if necessary.

### Metabolite Quantification and Data Normalization

Peaks were quantified using area-under-the-curve. For studies spanning multiple days, a data normalization step was performed to correct variation resulting from instrument inter-day tuning differences. Essentially, each compound was corrected in run-day blocks by registering the medians to equal one (1.00) and normalizing each data point proportionately (termed the "block correction". For studies that did not require more than one day of analysis, no normalization is necessary, other than for purposes of data visualization. In certain instances, biochemical data may have been normalized to an additional factor (e.g., cell counts, total protein as determined by Bradford assay, osmolality, etc.) to account for differences in metabolite levels due to differences in the amount of material present in each sample.

### Lipidomic analysis on plasma

Metabolon, Inc., Morrisville, USA performed the lipidomic analysis. Lipids were extracted from plasma in the presence of deuterated internal standards using an automated BUME extraction according to the method of Lofgren et al. [19]. The extracts were dried under nitrogen and reconstituted in ammonium acetate dichloromethane: methanol.

The extracts were transferred to vials for infusion-MS analysis, performed on a Shimadzu LC with nano PEEK tubing and the Sciex SelexIon-5500 QTRAP. The samples were analyzed via both positive and negative mode electrospray. The 5500 QTRAP was operated in MRM mode with a total of more than 1,100 MRMs. Individual lipid species were quantified by taking the ratio of the signal intensity of each target compound to that of its assigned internal standard, then multiplying by the concentration of internal standard added to the sample. Lipid class concentrations were calculated from the sum of all molecular species within a class, and fatty acid compositions were determined by calculating the proportion of each class comprised by individual fatty acids.

### Sphingomyelin determination in plasma

Sphingomyelin contents in human plasma were measured using a fluorimetric sphingomyelin assay kit (Abcam). Briefly, human plasma (diluted at 1:10) was treated with sphingomyelinase for two hours at 37°C, obtaining ceramide and phosphocholine. Then, samples were incubated with the AbRed fluorogenic dye that bound phosphocholine and analyzed with a spectrofluorimeter. The total sphingomyelins concentration was determined through the interpolation of the fluorescent emission with a standard curve, defined with known concentrations of sphingomyelin.

### Statistical analysis

Biomarker assays routinely report tens of thousands of individual results, be they proteins, metabolites, or gene expressions. The multitudinous nature of these biomarker observations complicates their use in classification or assessment problems, when the biomarkers must be related to some response variable. This is especially true if no *a priori* information relating specific biomarkers (or categories of biomarkers) to the response variable is available.

To this end, feature selection algorithms are commonly employed to identify which biomarker observations are most relevant to the response variable.

A template-oriented genetic algorithm has been employed.

### RESULTS

Patients enrolled in this study were divided in two groups based on the result of the ajmaline test. The number of patients included in each group (Control Group and Brugada Group) was 293, for a total of 586 subjects. In Table 4 the demographic features of each group are shown.

**TABLE 6**

| | Control Group | Brugada Group |
|---|---|---|
| Parameters | | |
| Male | 58.36 % | 68.47 % |
| Female | 41.63 % | 31.52 % |
| Age (years) | 33.03 ± 0.9135 | 40.92 ± 0.8009 |

### Metabolomics

The metabolomics dataset provided us with the normalized concentrations of 984 compounds in 586 subjects, including 293 cases and 293 controls. For the subsequent analysis, all the 169 xenobiotics were excluded. Thus, the metabolomics dataset was composed by 815 metabolites.

### Lipidomics

The lipidomics dataset provided us with the normalized concentrations of 1021 compounds in 586 subjects, including 293 cases and 293 controls. None of the lipids was excluded from the subsequent analysis.

### Transcriptomics

The transcriptomics analysis detected 15155 RNA molecules in 586 subjects, including 293 cases and 293 controls. None of the RNA was excluded from the subsequent analysis.

### Biomarkers determination

The biomarker discovery process was performed combining only the metabolomics, lipidomics and trancriptomics data.

The application of the template-oriented genetic algorithm highlighted the most important 15 to 60 features (including the age of the patient) useful for the diagnosis of Brugada Syndrome.

The 14 biomarkers set comprises 6 mRNA, 1 lipid and 7 metabolites as depicted in the following Table 7.

**TABLE 7: Identified 14 multi-omics biomarkers**

| | | | |
|---|---|---|---|
| dodecanedioate (C12:1-DC) | | | lipid |
| hypotaurine | | | metabolite |
| lysine | | | metabolite |
| γ-glutamylleucine | | | metabolite |
| 4-methoxyphenol sulfate | | | metabolite |
| phenol sulfate | | | metabolite |
| N-acetyl-2-aminooctanoate | | | metabolite |
| 2-aminooctanoate | | | metabolite |
| 'CAPN2' | Calpain 2 (CAPN2) | ENSG00000162909 | mRNA |
| 'HLA-DPA1' | Major Histocompatibility Complex, Class II, DP Alpha 1 (HLA-DPA1) | ENSG00000231389 | mRNA |
| 'HLA-DPB1' | Major Histocompatibility Complex, Class II, DP Beta 1 (HLA-DPB1) | ENSG00000223865 | mRNA |
| 'DCTN2' | Dynactin Subunit 2 (DCTN2) | ENSG00000175203 | mRNA |
| 'WDR86' | WD Repeat Domain 86 (WDR86) | ENSG00000187260 | mRNA |
| 'DGKD' | Diacylulycerol Kinase Delta (DGKD) | ENSG00000077044 | mRNA |

The 59 biomarkers set comprise 44 RNAs, 13 metabolites, 2 lipids as depicted in the following Table 8:

**TABLE 8: Identified 59 multi-omics biomarkers**

| **Biomarker** | **Ensemble ID** | **Type** |
|---|---|---|
| Acyl-CoA Synthetase Family Member 4 (AASDH) | ENSG00000157426 | mRNA |
| Ankyrin Repeat And Death Domain Containing 1A (ANKDD1A) | ENSG00000166839 | mRNA |
| Ankyrin Repeat Domain 49 (ANKRD49) | ENSG00000168876 | mRNA |
| Rho/Rac Guanine Nucleotide Exchange Factor 18 (ARHGEF18) | ENSG00000104880 | mRNA |
| BRICHOS Domain Containing 5 (BRICD5) | ENSG00000182685 | mRNA |
| BRCA1 Interacting Protein C-Terminal Helicase 1 (BRIP1) | ENSG00000136492 | mRNA |
| BRO1 Domain And CAAX Motif Containing (BROX) | ENSG00000162819 | mRNA |
| Glucocorticoid Receptor AF-1 Coactivator-1 (BTBD6) | ENSG00000184887 | mRNA |
| Chromosome 19 Open Reading Frame 79 (C12orf79) | ENSG00000257242 | Long noncoding RNA |
| Chromosome 19 Open Reading Frame 38 (C19orf38) | ENSG00000214212 | mRNA |
| Calcium/Calmodulin Dependent Protein Kinase Kinase 2 (CAMKK2) | ENSG00000110931 | mRNA |
| Calpain 2 (CAPN2) | ENSG00000162909 | mRNA |
| Chimerin I (CHN1) | ENSG00000128656 | mRNA |
| Cyclin-M3 (CNNM3) | ENSG00000168763 | mRNA |
| Cytochrome P450 Family 2 Subfamily S Member 1 (CYP2S1) | ENSG00000167600 | mRNA |
| Dynactin Subunit 2 (DCTN2) | ENSG00000175203 | mRNA |
| Diacylglycerol Kinase Delta (DGKD) | ENSG00000077044 | mRNA |
| ERBB Receptor Feedback Inhibitor 1 (ERRFI1) | ENSG00000116285 | mRNA |
| Retrotransposon Gag Like 8C (FAM127A) | ENSG00000134590 | mRNA |
| Glycoprotein, Alpha-Galactosyltransferase 1 (GGTA1P) | ENSG00000204136 | mRNA |
| Glutathione-Disulfide Reductase (GSR) | ENSG00000104687 | mRNA |
| HLA Complex Group 18 (HCG18) | ENSG00000231074 | mRNA |
| Histone Cluster 1 H1 Family Member C (HIST1H1C) | ENSG00000187837 | mRNA |
| Histone Cluster 1 H2B Family Member K (HIST1H2BK) | ENSG00000197903 | mRNA |
| Major Histocompatibility Complex, Class II, DP Alpha 1 (HLA-DPA1) | ENSG00000231389 | mRNA |
| Major Histocompatibility Complex, Class II, DP Beta 1 (HLA-DPB1) | ENSG00000223865 | mRNA |
| Insulin Like Growth Factor Binding Protein 3 (IGFBP3) | ENSG00000146674 | mRNA |
| Ras GTPase-Activating-Like Protein (IQGAP2) | ENSG00000145703 | mRNA |
| Lymphotoxin Beta (LTB) | ENSG00000227507 | mRNA |
| Methionine Adenosyltransferase 2° (MAT2A) | ENSG00000168906 | mRNA |
| Mitochondrial Ribosomal Protein L22 (MRPS22) | ENSG00000175110 | mRNA |
| Nucleosome Assembly Protein 1 Like 2 (NAP1L2) | ENSG00000186462 | mRNA |
| Peptidyl Arginine Deiminase 4 (PADI4) | ENSG00000159339 | mRNA |
| PDZ And LIM Domain 7 (PDLIM7) | ENSG00000196923 | mRNA |
| Platelet Activating Factor Receptor (PTAFR) | ENSG00000169403 | mRNA |
| RAN Binding Protein 6 (RANBP6) | ENSG00000137040 | mRNA |
| RNA, 7SL, Cytoplasmic 473, Pseudogene (RN7SL473P) | ENSG00000277452 | mRNA |
| RP11-429E11.3 | ENSG00000179253.3 | mRNA |
| RP11-4B16.1 | ENSG00000279433 | microRNA |
| Ribosomal Protein S20 (RPS20) | ENSG00000008988 | mRNA |
| Syntaxin 10 (STX10) | ENSG00000104915 | mRNA |
| T Cell Receptor Beta Variable 11-2 (TRBV11-2) | ENSG00000241657 | mRNA |
| WD Repeat Domain 86 (WDR86) | ENSG00000187260 | mRNA |
| Zwilch Kinetochore Protein (ZWILCH) | ENSG00000174442 | mRNA |
| Hypotaurine | | Metabolite |
| Lysine | | Metabolite |
| Glucoronate | | Metabolite |
| γ-glutamylleucine | | Metabolite |
| 3-(4-hydroxyphenyl)lactate | | Metabolite |
| Trans-4-hydroxyproline | | Metabolite |
| Phenol sulfate | | Metabolite |
| 5-methyluridine (ribothymidine) | | Metabolite |
| 2-aminooctanoate | | Metabolite |
| 4-methoxyphenol sulfate | | Metabolite |
| Fibrinopeptide A, phosphono-ser(3) | | Metabolite |
| N-acetyl-2-aminooctanoate | | Metabolite |
| Glyco-alpha-muricholate | | Metabolite |
| PI(16:0/18:1) | | Lipid |
| dodecanedioate(C12:1-DC) | | Lipid |

Then, a support vector machine classifier was employed to predict the Brugada Syndrome diagnosis in the study population, as described in the Material and Methods section.

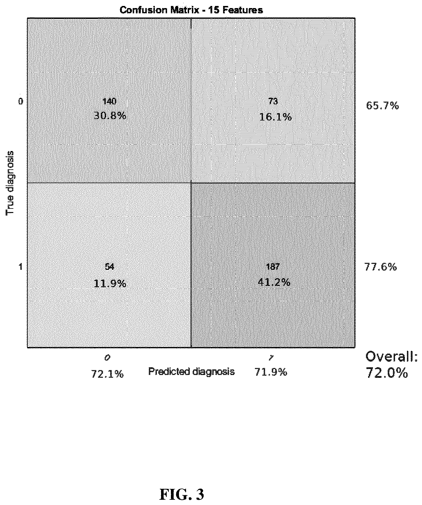

Figures 2 and 3 show a representative example of the results obtained from one such classifier, using all 15 features (14 biomarker + paten age). In this representative example, the classifier gives the following results:
- overall accuracy: 72.0%
- specificity: 65.7%
- sensitivity: 77.6%

The training population was composed by the 75% of our dataset, and the remaining 25% was used for the validation process. The dataset was randomly partitioned 200 times according to this method, and each random partition was used to train an independent support-vector machine model. The results of these 200 randomized models were compared to ensure consistency.

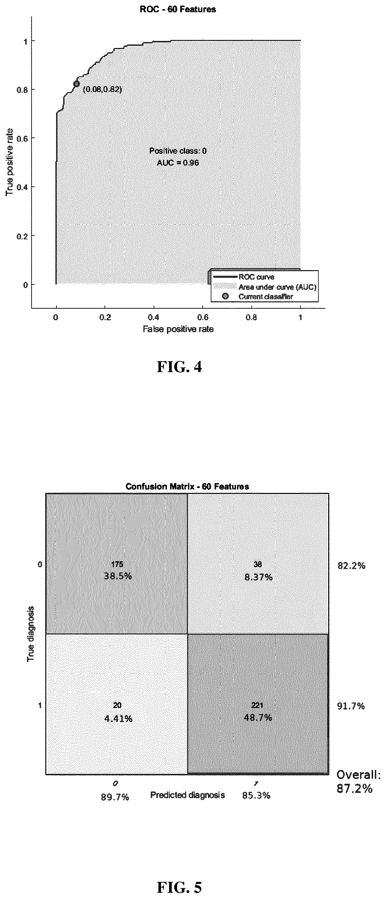

Figures 4 and 5 show a representative example of the results obtained from one such classifier, using all 60 features (59 biomarkers + patient age). In this representative example, the classifier gives the following results:
- overall accuracy: 87.2%
- specificity: 82.2%
- sensitivity: 91.7%

The terms "sensitivity" and "specificity" define the ability to correctly classify an individual, based on the biomarkers values detected, as having Brugada Syndrome or not. "Sensitivity" indicates the performance of the biomarker (s) with respect to correctly classifying individuals that have Brugada Syndrome. "Specificity" indicates the performance of the biomarkers with respect to correctly classifying individuals who do not have Brugada Syndrome. Thus, 84% specificity and 91% sensitivity for the panel of markers defined to test a set of control samples and Brugada Syndrome patients indicates that 84% of the control samples were correctly classified as control samples by the panel, and 91% of the Brugada syndrome samples were correctly classified as Brugada syndrome samples by the panel.

Finally, a comparison between the accuracy of the test carried out with less than 60 features and more than 60 features has been carried out. In general, it has been observed a linear increase in predictive ability between 15 and 60 features used (see

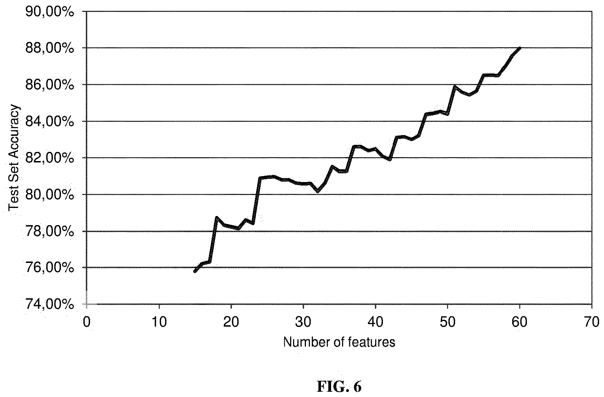

Figure 6), and a plateau between 60 and 150 (see Figure 7).

Thus, 60 is an optimal number of features to select, though at least 15 features may equivalently be used to make a meaningful diagnosis of the syndrome.

### REFERENCES

[1] Antzelevitch, C., et al. Circulation, 2005. 111(5): p. 659-70.
[2] Brugada, P. and J. Brugada. JAm Coll Cardiol, 1992. 20(6): p. 1391-6.
[3] Nademanee, K., et al. J Am Coll Cardiol, 2015. 66(18): p. 1976-86.
[4] Priori, S.G., et al. Eur Heart J, 2015. 36(41): p. 2793-867.
[5] Mellor, G., et al. Circ Arrhythm Electrophysiol, 2014. 7(6): p. 1078-83.
[6] Nademanee, K., et al. Circulation, 1997. 96(8): p. 2595-600.
[7] Tan, H.L., et al. Circulation, 2005. 112(2): p. 207-13.
[8] Pappone, C., et al. J Am Coll Cardiol, 2018. 71(15): p. 1631-1646.
[9] Chen, Q., et al. Nature, 1998. 392(6673): p. 293-6.
[10] Kapplinger, J.D., et al. Heart Rhythm, 2010. 7(1): p. 33-46.
[11] Antzelevitch, C. and B. Patocskai. Curr Probl Cardiol, 2016. 41(1): p. 7-57.
[12] Mango, R., et al. Circ J, 2016. 80(4): p. 938-49.
[13] Pappone, C., M. Monasky, and G. Ciconte. Eur Heart J Suppl, 2019. 21(Suppl B): p. B61-B66.
[14] Yar, S., M.M. Monasky, and R.J. Solaro. Pflugers Arch, 2014. 466(6): p. 1189-97.
[15] Monasky, M.M., et al. Front Physiol, 2017. 8: p. 1056.
[16] Monasky, M.M., et al. Front Physiol, 2018. 9: p. 1088.
[17] Letsas, K.P., et al. Pacing Clin Electrophysiol, 2017. 40(12): p. 1332-1345.
[18] Hofman, N., et al. Circulation, 2013. 128(14): p. 1513-21.
[19] Lofgren et al. J Lipid Res 2012; 53(8): p.1690-700.

## Claims

1. A set of biomarkers comprising at least:
i) the subset of 6 mRNA listed in the following Table:
| **Name** | **Ensemble ID** |
|---|---|
| Calpain 2 (CAPN2) | ENSG00000162909 |
| Dynactin Subunit 2 (DCTN2) | ENSG00000175203 |
| Diacylglycerol Kinase Delta (DGKD) | ENSG00000077044 |
| Major Histocompatibility Complex, Class II, DP Alpha 1 (HLA-DPA1) | ENSG00000231389 |
| Major Histocompatibility Complex, Class II, DP Beta 1 (HLA-DPB1) | ENSG00000223865 |
| WD Repeat Domain 86 (WDR86) | ENSG00000187260 |
ii) the subset of the following 7 metabolites:
- 2-aminooctanoate
- 4-methoxyphenol sulfate
- hypotaurine
- lysine
- N-acetyl-2-aminooctanoate
- phenol sulfate
- γ-glutamylleucine; and
iii) the lipid dodecanedioate (C12:1-DC);
for the diagnosis of Brugada Syndrome in a human being.

2. A set of biomarkers according to claim 1, further comprising one or more of the following additional biomarkers:
i) RNA selected from the group consisting of:
| **Name** | **Ensemble ID** | **Type** |
|---|---|---|
| Acyl-CoA Synthetase Family Member 4 (AASDH) | ENSG00000157426 | mRNA |
| Ankyrin Repeat And Death Domain Containing 1A (ANKDD1A) | ENSG00000166839 | mRNA |
| Ankyrin Repeat Domain 49 (ANKRD49) | ENSG00000168876 | mRNA |
| Rho/Rac Guanine Nucleotide Exchange Factor 18 (ARHGEF18) | ENSG00000104880 | mRNA |
| BRICHOS Domain Containing 5 (BRICD5) | ENSG00000182685 | mRNA |
| BRCA1 Interacting Protein C-Terminal Helicase 1 (BRIP1) | ENSG00000136492 | mRNA |
| BRO1 Domain And CAAX Motif Containing (BROX) | ENSG00000162819 | mRNA |
| Glucocorticoid Receptor AF-1 Coactivator-1 (BTBD6) | ENSG00000184887 | mRNA |
| Chromosome 19 Open Reading Frame 79 (C12orf79) | ENSG00000257242 | Long noncoding RNA |
| Chromosome 19 Open Reading Frame 38 (C19orf38) | ENSG00000214212 | mRNA |
| Calcium/Calmodulin Dependent Protein Kinase Kinase 2 (CAMKK2) | ENSG00000110931 | mRNA |
| Chimerin I (CHN1) | ENSG00000128656 | mRNA |
| Cyclin-M3 (CNNM3) | ENSG00000168763 | mRNA |
| Cytochrome P450 Family 2 Subfamily S Member 1 (CYP2S1) | ENSG00000167600 | mRNA |
| ERBB Receptor Feedback Inhibitor 1 (ERRFI1) | ENSG00000116285 | mRNA |
| Retrotransposon Gag Like 8C (FAM127A) | ENSG00000134590 | mRNA |
| Glycoprotein, Alpha-Galactosyltransferase (GGTA1P) | ENSG00000204136 | mRNA |
| Glutathione-Disulfide Reductase (GSR) | ENSG00000104687 | mRNA |
| HLA Complex Group 18 (HCG18) | ENSG00000231074 | mRNA |
| Histone Cluster 1 H1 Family Member C (HIST1H1C) | ENSG00000187837 | mRNA |
| Histone Cluster 1 H2B | ENSG00000197903 | mRNA |
| Family Member K (HIST1H2BK) | | |
| Insulin Like Growth Factor Binding Protein 3 (IGFBP3) | ENSG00000146674 | mRNA |
| Ras GTPase-Activating-Like Protein (IQGAP2) | ENSG00000145703 | mRNA |
| Lymphotoxin Beta (LTB) | ENSG00000227507 | mRNA |
| Methionine Adenosyltransferase 2A (MAT2A) | ENSG00000168906 | mRNA |
| Mitochondrial Ribosomal Protein L22 (MRPS22) | ENSG00000175110 | mRNA |
| Nucleosome Assembly Protein 1 Like 2 (NAP1L2) | ENSG00000186462 | mRNA |
| Peptidyl Arginine Deiminase 4 (PADI4) | ENSG00000159339 | mRNA |
| PDZ And LIM Domain 7 (PDLIM7) | ENSG00000196923 | mRNA |
| Platelet Activating Factor Receptor (PTAFR) | ENSG00000169403 | mRNA |
| RAN Binding Protein 6 (RANBP6) | ENSG00000137040 | mRNA |
| RNA, 7SL, Cytoplasmic 473, Pseudogene (RN7SL473P) | ENSG00000277452 | mRNA |
| RP11-429E11.3 | ENSG00000179253.3 | mRNA |
| RP11-4B16.1 | ENSG00000279433 | microRNA |
| Ribosomal Protein S20 (RPS20) | ENSG00000008988 | mRNA |
| Syntaxin 10 (STX10) | ENSG00000104915 | mRNA |
| T Cell Receptor Beta Variable 11-2 (TRBV11-2) | ENSG00000241657 | mRNA |
| Zwilch Kinetochore Protein (ZWILCH) | ENSG00000174442 | mRNA |
ii) metabolite selected from the group consisting of:
- 5-methyluridine (ribothymidine)
- fibrinopeptide A, phosphono-ser(3)
- glyco-alpha-muricholate;
- 3-(4-hydroxyphenyl)lactate
- glucoronate
- Trans-4-hydroxyproline;
iii) phosphatidylinositol (16:0/18:1); or a combination thereof.

3. A set of biomarkers according to claim 2, comprising:
i) the subset of 44 RNAs listed in the following Table:
| **Name** | **Ensemble ID** | **Type** |
|---|---|---|
| Acyl-CoA Synthetase Family Member 4 (AASDH) | ENSG00000157426 | mRNA |
| Ankyrin Repeat And Death Domain Containing 1A (ANKDD1A) | ENSG00000166839 | mRNA |
| Ankyrin Repeat Domain 49 (ANKRD49) | ENSG00000168876 | mRNA |
| Rho/Rac Guanine Nucleotide Exchange Factor 18 (ARHGEF18) | ENSG00000104880 | mRNA |
| BRICHOS Domain Containing 5 (BRICD5) | ENSG00000182685 | mRNA |
| BRCA1 Interacting Protein C-Terminal Helicase 1 (BRIP1) | ENSG00000136492 | mRNA |
| BRO1 Domain And CAAX Motif Containing (BROX) | ENSG00000162819 | mRNA |
| Glucocorticoid Receptor AF-1 Coactivator-1 (BTBD6) | ENSG00000184887 | mRNA |
| Chromosome 19 Open Reading Frame 79 (C12orf79) | ENSG00000257242 | Long noncoding RNA |
| Chromosome 19 Open Reading Frame 38 (C19orf38) | ENSG00000214212 | mRNA |
| Calcium/Calmodulin Dependent Protein Kinase Kinase 2 (CAMKK2) | ENSG00000110931 | mRNA |
| Calpain 2 (CAPN2) | ENSG00000162909 | mRNA |
| Chimerin I (CHN1) | ENSG00000128656 | mRNA |
| Cyclin-M3 (CNNM3) | ENSG00000168763 | mRNA |
| Cytochrome P450 Family 2 Subfamily S Member 1 (CYP2S1) | ENSG00000167600 | mRNA |
| Dynactin Subunit 2 (DCTN2) | ENSG00000175203 | mRNA |
| Diacylglycerol Kinase Delta (DGKD) | ENSG00000077044 | mRNA |
| ERBB Receptor Feedback Inhibitor 1 (ERRFI1) | ENSG00000116285 | mRNA |
| Retrotransposon Gag Like 8C (FAM127A) | ENSG00000134590 | mRNA |
| Glycoprotein, Alpha-Galactosyltransferase (GGTA1P) | ENSG00000204136 | mRNA |
| Glutathione-Disulfide Reductase (GSR) | ENSG00000104687 | mRNA |
| HLA Complex Group 18 (HCG18) | ENSG00000231074 | mRNA |
| Histone Cluster 1 H1 Family Member C (HIST1H1C) | ENSG00000187837 | mRNA |
| Histone Cluster 1 H2B Family Member K (HIST1H2BK) | ENSG00000197903 | mRNA |
| Major Histocompatibility Complex, Class II, DP Alpha 1 (HLA-DPA1) | ENSG00000231389 | mRNA |
| Major Histocompatibility Complex, Class II, DP Beta 1 (HLA-DPB1) | ENSG00000223865 | mRNA |
| Insulin Like Growth Factor Binding Protein 3 (IGFBP3) | ENSG00000146674 | mRNA |
| Ras GTPase-Activating-Like Protein (IQGAP2) | ENSG00000145703 | mRNA |
| Lymphotoxin Beta (LTB) | ENSG00000227507 | mRNA |
| Methionine Adenosyltransferase 2A (MAT2A) | ENSG00000168906 | mRNA |
| Mitochondrial Ribosomal Protein L22 (MRPS22) | ENSG00000175110 | mRNA |
| Nucleosome Assembly Protein 1 Like 2 (NAP1L2) | ENSG00000186462 | mRNA |
| Peptidyl Arginine Deiminase 4 (PADI4) | ENSG00000159339 | mRNA |
| PDZ And LIM Domain 7 (PDLIM7) | ENSG00000196923 | mRNA |
| Platelet Activating Factor Receptor (PTAFR) | ENSG00000169403 | mRNA |
| RAN Binding Protein 6 (RANBP6) | ENSG00000137040 | mRNA |
| RNA, 7SL, Cytoplasmic 473, Pseudogene (RN7SL473P) | ENSG00000277452 | mRNA |
| RP11-429E11.3 | ENSG00000179253.3 | mRNA |
| RP11-4B16.1 | ENSG00000279433 | microRNA |
| Ribosomal Protein S20 (RPS20) | ENSG00000008988 | mRNA |
| Syntaxin 10 (STX10) | ENSG00000104915 | mRNA |
| T Cell Receptor Beta Variable 11-2 (TRBV11-2) | ENSG00000241657 | mRNA |
| WD Repeat Domain 86 (WDR86) | ENSG00000187260 | mRNA |
| Zwilch Kinetochore Protein (ZWILCH) | ENSG00000174442 | mRNA |
ii) the subset of the following 13 metabolites:
- 2-aminooctanoate
- 3-(4-hydroxyphenyl)lactate
- 4-methoxyphenol sulfate
- hypotaurine
- lysine
- glucoronate
- Trans-4-hydroxyproline
- N-acetyl-2-aminooctanoate
- phenol sulfate
- γ-glutamylleucine
- 5-methyluridine (ribothymidine)
- fibrinopeptide A, phosphono-ser(3)
- glyco-alpha-muricholate;
and iii) the subset of the following lipids:
- dodecanedioate (C12:1-DC)
- phosphatidylinositol (16:0/18:1);
for the diagnosis of Brugada Syndrome in a human being.

4. An in vitro method for detecting the presence of the occurrence of one of the set of biomarkers according to anyone of claim 1-3 in a biological sample of a human being, which comprises the following steps:
a) detecting the presence of at least 6 up to 44 RNAs of subset i) as defined in claim 1 and 3 by an assay selected from the group comprising mRNA microarray detection, Real-Time PCR, Droplet Digital PCR or hybridization-based assays, and branched DNA (bDNA) technology (i.e. QuantiGene^{®} assay), or a combination thereof; and
b) detection of at least 7 up to 13 of the metabolites of subset ii) as defined in claim 1 and 3 and one or both the lipids of subset iii) as defined in claim 1 and 3 by an assay such as one selected from the group comprising MS, GC-MS, fluorimetric or colorimetric assays, NMR, spectroscopy with nanoprobes, Enzyme-Linked oligonucleotide assays, or a combination thereof;
wherein the positive detection of all the biomarkers compared to a normal control allows the identification of patients affected by Brugada Syndrome.

5. An in vitro method for measuring the concentration of one of the set of biomarkers according to anyone of claim 1-3 in a biological sample of a human being, comprising the following steps:
a) quantitative determination of at least 6 up to 44 RNAs of subset i) as defined in claim 1 and 3 by a quantitative technique such as quantitative PCR, preferably by Real Time-PCR, Droplet Digital PCR, Quantigene assay, or a combination thereof; and
b) quantitative determination of at least 7 up to 13 of the metabolites of subset ii) as defined in claim 1 and 3 by a technique selected between HPLC, MS, NMR analysis, ELONA, or a combination thereof; and
c) quantitative determination of one or both the lipids of subset iii) as defined in claim 1 and 3 by a technique selected between HPLC, MS, NMR analysis, or a combination thereof;
wherein an increase of the concentration of the biomarkers compared to a normal control indicates a diagnosis of Brugada Syndrome.

6. An in vitro method according to anyone of the claims 4-5, wherein the biological sample is selected from the group consisting of whole blood, plasma, serum, peripheral blood and PBMCs.

7. An in vitro method according to anyone of the claims 4-6, wherein the human being is asymptomatic.

8. An in vitro method according to anyone of the claims 4-7, wherein the human being is at high risk for Brugada Syndrome due to family history, previous events of heart atrial and/or ventricular fibrillation, diabetes or obesity.

9. An in vitro method according to anyone of the claims 4-8, wherein the human being is about 40 years old or younger.

10. Use of a kit comprising oligonucleotides that are at least 85, 90, 95, 96, 97, 98, 99 or 100% complementary to each of the RNAs of subset i) according to anyone of claim 1-3, wherein said oligonucleotides are primers or probes optionally attached to a solid support, for the diagnosis of Brugada Syndrome in a human being.

## Patentansprüche

1. Menge von Biomarkern, umfassend mindestens:
i) die in der folgenden Tabelle aufgeführte Teilmenge von 6 mRNAs:
| **Name** | **Ensemble-ID** |
|---|---|
| Calpain 2 (CAPN2) | ENSG00000162909 |
| Dvnactin-Untereinheit 2 (DCTN2) | ENSG00000175203 |
| Diacylglycerol-Kinase-Delta (DGKD) | ENSG00000077044 |
| Haupthistokompatibilitätskomplex, Klasse II, DP Alpha 1 (HLA-DPA1) | ENSG00000231389 |
| Haupthistokompatibilitätskomplex, Klasse II, DP Beta 1 (HLA-DPB1) | ENSG00000223865 |
| WD-Wiederholungsdomäne 86 (WDR86) | ENSG00000187260 |
ii) die Teilmenge der folgenden 7 Metaboliten:
- 2-Aminooctanoat
- 4-Methoxyphenolsulfat
- Hypotaurin
- Lysin
- N-Acetyl-2-aminooctanoat
- Phenolsulfat
- γ-Glutamylleucin;
und
iii) das Lipiddodecandioat (C12:1-DC);
für die Diagnose des Brugada-Syndroms bei einem Menschen.

2. Menge von Biomarkern nach Anspruch 1, ferner umfassend einen oder mehrere der folgenden zusätzlichen Biomarker:
i) RNA ausgewählt aus der Gruppe, bestehend aus:
| **Name** | **Ensemble-ID** | **Typ** |
|---|---|---|
| Acyl-CoA-Synthetase-Familienmitglied 4 (AASDH) | ENSG00000157426 | mRNA |
| Ankyrin-Wiederholungs- und Todesdomäne Enthaltend 1A (ANKDD1A) | ENSG00000166839 | mRNA |
| Ankyrin-Wiederholungsdomäne 49 (ANKRD49) | ENSG00000168876 | mRNA |
| Rho/Rac-Guanin-Nukleotid-Austauschfaktor 18 (ARHGEF18) | ENSG00000104880 | mRNA |
| BRICHOS-Domäne Enthaltend 5 (BRICD5) | ENSG00000182685 | mRNA |
| BRCA1-Interaktives Protein C-Terminale Helicase 1 (BRIP1) | ENSG00000136492 | mRNA |
| BRO1-Domäne und CAAX-Motiv Enthaltend (BROX) | ENSG00000162819 | mRNA |
| Glukokortikoidrezeptor AF-1 Coaktivator-1 (BTBD6) | ENSG00000184887 | mRNA |
| Chromosom 19 Offener Leserahmen 79 (C12orf79) | ENSG00000257242 | Langes nicht kodierendes RNA |
| Chromosom 19 Offener Leserahmen 38 (C19orf38) | ENSG00000214212 | mRNA |
| Calcium/Calmodulin-abhängige Proteinkinase Kinase 2 (CAMKK2) | ENSG00000110931 | mRNA |
| Chimerin I (CHN1) | ENSG00000128656 | mRNA |
| Cyclin-M3 (CNNM3) | ENSG00000168763 | mRNA |
| Cytochrom P450 Familie 2 Unterfamilie S Mitglied 1 (CYP2S1) | ENSG00000167600 | mRNA |
| ERBB-Rezeptor-Feedback-Inhibitor 1 (ERRFI1) | ENSG00000116285 | mRNA |
| Retrotransposon-Gag Wie 8C (FAM127A) | ENSG00000134590 | mRNA |
| Glykoprotein, Alpha-Galactosyltransferase (GGTA1P) | ENSG00000204136 | mRNA |
| Glutathion-Disulfid-Reduktase (GSR) | ENSG00000104687 | mRNA |
| HLA-Komplex-Gruppe 18 (HCG18) | ENSG00000231074 | mRNA |
| Histon-Cluster 1 H1 Familienmitglied C (HIST1H1C) | ENSG00000187837 | mRNA |
| Histon-Cluster 1 H2B Familienmitglied C (HIST1H2BK) | ENSG00000197903 | mRNA |
| Insulin-ähnlicher Wachstumsfaktor Bindungsprotein 3 (IGFBP3) | ENSG00000146674 | mRNA |
| Ras-GTPase-aktivierendes-ähnliches Protein (IQGAP2) | ENSG00000145703 | mRNA |
| Lymphotoxin-Beta (LTB) | ENSG00000227507 | mRNA |
| Methionin-Adenosyltransferase 2A (MAT2A) | ENSG00000168906 | mRNA |
| Mitochondriales Ribosomenprotein L22 (MRPS22) | ENSG00000175110 | mRNA |
| Nukleosom-Assemblierungsprotein 1 Wie 2 (NAP1L2) | ENSG00000186462 | mRNA |
| Peptidyl-Arginin-Deiminase 4 (PADI4) | ENSG00000159339 | mRNA |
| PDZ- und LIM-Domäne 7 (PDLIM7) | ENSG00000196923 | mRNA |
| Thrombozytenaktivierungsfaktor-Rezeptor (PTAFR) | ENSG00000169403 | mRNA |
| RAN-Bindungsprotein 6 (RANBP6) | ENSG00000137040 | mRNA |
| RNA, 7SL, Zytoplasmatisch 473, Pseudogen (RN7SL473P) | ENSG00000277452 | mRNA |
| RP11-429E11.3 | ENSG00000179253.3 | mRNA |
| RP11-4B16.1 | ENSG00000279433 | MikroRNA |
| Ribosomales Protein S20 (RPS20) | ENSG00000008988 | mRNA |
| Syntaxin 10 (STX10) | ENSG00000104915 | mRNA |
| T-Zell-Rezeptor Beta-Variable 11-2 (TRBV11-2) | ENSG00000241657 | mRNA |
| Zwilch-Kinetochore-Protein (ZWILCH) | ENSG00000174442 | mRNA |
ii) Metabolit ausgewählt aus der Gruppe bestehend aus:
- 5-Methyluridin (Ribothymidin)
- Fibrinopeptid A, Phosphono-ser(3)
- Glyco-alpha-muricholat;
- 3-(4-Hydroxyphenyl)lactat
- Glucoronat
- Trans-4-Hydroxyprolin;
iii) Phosphatidylinositol (16:0/18:1); oder eine Kombination davon.

3. Menge von Biomarkern nach Anspruch 2, umfassend:
i) die in der folgenden Tabelle aufgeführte Teilmenge von 44 RNAs:
| **Name** | **Ensemble-ID** | **Typ** |
|---|---|---|
| Acyl-CoA-Synthetase-Familienmitglied 4 (AASDH) | ENSG00000157426 | mRNA |
| Ankyrin-Wiederholungs- und Todesdomäne Enthaltend 1A (ANKDD1A) | ENSG00000166839 | mRNA |
| Ankyrin-Wiederholungsdomäne 49 (ANKRD49) | ENSG00000168876 | mRNA |
| Rho/Rac-Guanin-Nukleotid-Austauschfaktor 18 (ARHGEF18) | ENSG00000104880 | mRNA |
| BRICHOS-Domäne Enthaltend 5 (BRICD5) | ENSG00000182685 | mRNA |
| BRCA1-Interaktives Protein C-Terminale Helicase 1 (BRIP1) | ENSG00000136492 | mRNA |
| BRO1-Domäne und CAAX-Motiv Enthaltend (BROX) | ENSG00000162819 | mRNA |
| Glukokortikoidrezeptor AF-1 Coaktivator-1 (BTBD6) | ENSG00000184887 | mRNA |
| Chromosom 19 Offener Leserahmen 79 (C12orf79) | ENSG00000257242 | Langes nicht kodierendes RNA |
| Chromosom 19 Offener Leserahmen 38 (C19orf38) | ENSG00000214212 | mRNA |
| Calcium/Calmodulin-abhängige Proteinkinase Kinase 2 (CAMKK2) | ENSG00000110931 | mRNA |
| Calpain 2 (CAPN2) | ENSG00000162909 | mRNA |
| Chimerin I (CHN1) | ENSG00000128656 | mRNA |
| Cvclin-M3 (CNNM3) | ENSG00000168763 | mRNA |
| Cytochrom P450 Familie 2 Unterfamilie S Mitglied 1 (CYP2S1) | ENSG00000167600 | mRNA |
| Dvnactin-Untereinheit 2 (DCTN2) | ENSG00000175203 | mRNA |
| Diacylglycerol-Kinase-Delta (DGKD) | ENSG00000077044 | mRNA |
| ERBB-Rezeptor-Feedback-Inhibitor 1 (ERRFI1) | ENSG00000116285 | mRNA |
| Retrotransposon-Gag Wie 8C (FAM127A) | ENSG00000134590 | mRNA |
| Glykoprotein, Alpha-Galactosyltransferase (GGTA1P) | ENSG00000204136 | mRNA |
| Glutathion-Disulfid-Reduktase (GSR) | ENSG00000104687 | mRNA |
| HLA-Komplex-Gruppe 18 (HCG18) | ENSG00000231074 | mRNA |
| Histon-Cluster 1 H1 Familienmitglied C (HIST1H1C) | ENSG00000187837 | mRNA |
| Histon-Cluster 1 H2B Familienmitglied C (HIST1H2BK) | ENSG00000197903 | mRNA |
| Haupthistokompatibilitätskomplex, Klasse II, DP Alpha 1 (HLA-DPA1) | ENSG00000231389 | mRNA |
| Haupthistokompatibilitätskomplex, Klasse II, DP Beta 1 (HLA-DPB1) | ENSG00000223865 | mRNA |
| Insulin-ähnlicher Wachstumsfaktor Bindungsprotein 3 (IGFBP3) | ENSG00000146674 | mRNA |
| Ras-GTPase-aktivierendes-ähnliches Protein (IQGAP2) | ENSG00000145703 | mRNA |
| Lymphotoxin-Beta (LTB) | ENSG00000227507 | mRNA |
| Methionin-Adenosyltransferase 2A (MAT2A) | ENSG00000168906 | mRNA |
| Mitochondriales Ribosomenprotein L22 (MRPS22) | ENSG00000175110 | mRNA |
| Nukleosom-Assemblierungsprotein 1 Wie 2 (NAP1L2) | ENSG00000186462 | mRNA |
| Peptidyl-Arginin-Deiminase 4 (PADI4) | ENSG00000159339 | mRNA |
| PDZ- und LIM-Domäne 7 (PDLIM7) | ENSG00000196923 | mRNA |
| Thrombozytenaktivierungsfaktor-Rezeptor (PTAFR) | ENSG00000169403 | mRNA |
| RAN-Bindungsprotein 6 (RANBP6) | ENSG00000137040 | mRNA |
| RNA, 7SL, Zytoplasmatisch 473, Pseudogen (RN7SL473P) | ENSG00000277452 | mRNA |
| RP11-429E11.3 | ENSG00000179253.3 | mRNA |
| RP11-4B16.1 | ENSG00000279433 | MikroRNA |
| Ribosomales Protein S20 (RPS20) | ENSG00000008988 | mRNA |
| Syntaxin 10 (STX10) | ENSG00000104915 | mRNA |
| T-Zell-Rezeptor Beta-Variable 11-2 (TRBV11-2) | ENSG00000241657 | mRNA |
| WD-Wiederholungsdomäne 86 (WDR86) | ENSG00000187260 | mRNA |
| Zwilch-Kinetochore-Protein (ZWILCH) | ENSG00000174442 | mRNA |
ii) die Teilmenge der folgenden 13 Metaboliten:
- 2-Aminooctanoat
- 3-(4-Hydroxyphenyl)lactat
- 4-Methoxyphenolsulfat
- Hypotaurin
- Lysin
- Glucoronat
- Trans-4-Hydroxyprolin
- N-Acetyl-2-aminooctanoat
- Phenolsulfat
- γ-Glutamylleucin
- 5-Methyluridin (Ribothymidin)
- Fibrinopeptid A, Phosphono-ser(3)
- Glyco-alpha-muricholat;
und iii) die Teilmenge der folgenden Lipide:
- Dodecandioat (C12:1-DC)
- Phosphatidylinositol (16:0/18:1);
für die Diagnose des Brugada-Syndroms bei einem Menschen.

4. In vitro-Verfahren zum Nachweisen des Vorhandenseins des Auftretens von einem aus der Menge von Biomarkern nach einem der Ansprüche 1-3 in einer biologischen Probe eines Menschen, das die folgenden Schritte umfasst:
a) Nachweisen des Vorhandenseins von mindestens 6 bis zu 44 RNAs der Teilmenge i), wie in Anspruch 1 und 3 definiert, durch einen Assay, ausgewählt aus der Gruppe, umfassend mRNA-Microarray-Nachweis, Real-Time-PCR, Droplet-Digital-PCR oder Hybridisierungs-basierte Assays und verzweigte DNA (bDNA)-Technologie (d. h. QuantiGene ^{®}-Assay) oder eine Kombination davon; und
b) Nachweis von mindestens 7 bis zu 13 der Metaboliten der Teilmenge ii), wie in Anspruch 1 und 3 definiert, und eines oder beider der Lipide der Teilmenge iii), wie in Anspruch 1 und 3 definiert, durch einen Assay, wie beispielsweise einen ausgewählt aus der Gruppe umfassend MS, GC-MS, fluorimetrische oder kolorimetrische Assays, NMR, Spektroskopie mit Nanosonden, Enzymverknüpfte Oligonukleotid-Assays oder eine Kombination davon;
wobei der positive Nachweis aller Biomarker im Vergleich zu einer normalen Kontrolle die Identifizierung von Patienten mit Brugada-Syndrom ermöglicht.

5. In vitro-Verfahren zum Messen der Konzentration von einem aus der Menge von Biomarkern nach einem der Ansprüche 1-3 in einer biologischen Probe eines Menschen, das die folgenden Schritte umfasst:
a) quantitative Bestimmung von mindestens 6 bis zu 44 RNAs der Teilmenge i), wie in Anspruch 1 und 3 definiert, durch eine quantitative Technik wie quantitative PCR, vorzugsweise durch Real Time-PCR, Droplet Digital PCR, Quantigen-Assay oder eine Kombination davon; und
b) quantitative Bestimmung von mindestens 7 bis zu 13 der Metaboliten der Teilmenge ii), wie in Anspruch 1 und 3 definiert, durch eine Technik, ausgewählt aus HPLC, MS, NMR-Analyse, ELONA oder einer Kombination davon; und
c) quantitative Bestimmung eines oder beider Lipide der Teilmenge iii), wie in Anspruch 1 und 3 definiert, durch eine Technik, ausgewählt aus HPLC-, MS-, NMR-Analyse oder einer Kombination davon;
wobei ein Anstieg der Konzentration der Biomarker im Vergleich zu einer normalen Kontrolle auf eine Diagnose des Brugada-Syndroms hinweist.

6. In vitro-Verfahren nach einem der Ansprüche 4-5, wobei die biologische Probe aus der Gruppe ausgewählt ist, die aus Vollblut, Plasma, Serum, peripherem Blut und PBMCs besteht.

7. In-vitro-Verfahren nach einem der Ansprüche 4-6, wobei der Mensch asymptomatisch ist.

8. In-vitro-Verfahren nach einem der Ansprüche 4-7, wobei der Mensch ein hohes Risiko für das Brugada-Syndrom aufgrund von Familienanamnese, früheren Ereignissen von Vorhof- und/oder Kammerflimmern, Diabetes oder Fettleibigkeit hat.

9. In-vitro-Verfahren nach einem der Ansprüche 4-8, wobei der Mensch etwa 40 Jahre alt oder jünger ist.

10. Verwendung eines Kits, umfassend Oligonukleotide, die mindestens 85, 90, 95, 96, 97, 98, 99 oder 100% komplementär zu jedem der RNAs der Teilmenge i) sind, nach einem der Ansprüche 1-3, wobei die Oligonukleotide Primer oder Sonden sind, die optional an einen festen Träger gebunden sind, zur Diagnose des Brugada-Syndroms bei einem Menschen.

## Revendications

1. Ensemble de biomarqueurs comprenant au moins :
i) le sous-ensemble de 6 ARNm énumérés dans le tableau suivant :
| **Nom** | **ID de l'ensemble** |
|---|---|
| Calpaïne 2 (CAPN2) | ENSG00000162909 |
| Sous-unité 2 de la dynactine (DCTN2) | ENSG00000175203 |
| Diacylglycérol kinase delta (DGKD) | ENSG00000077044 |
| Complexe majeur d'histocompatibilité, classe II, DP Alpha 1 (HLA-DPA1) | ENSG00000231389 |
| Complexe majeur d'histocompatibilité, classe II, DP Bêta 1 (HLA-DPB1) | ENSG00000223865 |
| Domaine de répétition WD 86 (WDR86) | ENSG00000187260 |
ii) le sous-ensemble des 7 métabolites suivants :
- 2-aminooctanoate
- sulfate de 4-méthoxyphénol
- hypotaurine
- lysine
- N-acétyl-2-aminooctanoate
- sulfate de phénol
- γ-glutamylleucine ;
et
iii) le lipide dodécanedioate (C12:1-DC) ;
pour le diagnostic du syndrome de Brugada chez un être humain.

2. Ensemble de biomarqueurs selon la revendication 1, comprenant en outre un ou plusieurs des biomarqueurs supplémentaires suivants :
i) ARN choisi dans le groupe constitué par :
| **Nom** | **ID de l'ensemble** | **Type** |
|---|---|---|
| Membre 4 de la famille des Acyl-CoA synthétases (AASDH) | ENSG00000157426 | ARNm |
| Répétition de l'ankyrine et domaine de la mort contenant 1A (ANKDD1A) | ENSG00000166839 | ARNm |
| Domaine de répétition de l'ankvrine 49 (ANKRD49) | ENSG00000168876 | ARNm |
| Facteur d'échange de nucléotides de guanine Rho/Rac 18 (ARHGEF18) | ENSG00000104880 | ARNm |
| Domaine BRICHOS contenant 5 (BRICD5) | ENSG00000182685 | ARNm |
| Protéine d'interaction BRCA1 avec la C-terminal hélicase 1 (BRIP1) | ENSG00000136492 | ARNm |
| Domaine BRO1 et contenant le motif CAAX (BROX) | ENSG00000162819 | ARNm |
| Coactivateur-1 du récepteur AF-1 des glucocorticoïdes (BTBD6) | ENSG00000184887 | ARNm |
| Cadre de lecture ouvert 79 du chromosome 19 (C12orf79) | ENSG00000257242 | ARN long non codant |
| Cadre de lecture ouvert 38 du chromosome 19 (C19orf38) | ENSG00000214212 | ARNm |
| Protéine kinase dépendante du calcium et de la calmoduline kinase 2 (CAMKK2) | ENSG00000110931 | ARNm |
| Chimérine I (CHN1) | ENSG00000128656 | ARNm |
| Cvcline-M3 (CNNM3) | ENSG00000168763 | ARNm |
| Membre 1 de la sous-famille s de la famille 2 du cytochrome P450 (CYP2S1) | ENSG00000167600 | ARNm |
| Inhibiteur de rétroaction du récepteur ERBB 1 (ERRFI1) | ENSG00000116285 | ARNm |
| Rétrotransposon gag like 8C (FAM127A) | ENSG00000134590 | ARNm |
| Glycoprotéine, alpha-galactosyltransférase (GGTA1P) | ENSG00000204136 | ARNm |
| Glutathion-disulfure réductase (GSR) | ENSG00000104687 | ARNm |
| Groupe 18 du complexe HLA (HCG18) | ENSG00000231074 | ARNm |
| Membre C de la famille du groupe d'histones 1 H1 (HIST1H1C) | ENSG00000187837 | ARNm |
| Membre K de la famille du groupe d'histones 1 H2B (HIST1H2BK) | ENSG00000197903 | ARNm |
| Protéine de liaison du facteur de croissance analogue à l'insuline 3 (IGFBP3) | ENSG00000146674 | ARNm |
| Protéine Ras GTPase-activating-like (IQGAP2) | ENSG00000145703 | ARNm |
| Lymphotoxine bêta (LTB) | ENSG00000227507 | ARNm |
| Méthionine adénosyltransférase 2A (MAT2A) | ENSG00000168906 | ARNm |
| Protéine ribosomale mitochondriale L22 (MRPS22) | ENSG00000175110 | ARNm |
| Protéine d'assemblage du nucléosome 1 like 2 (NAP1L2) | ENSG00000186462 | ARNm |
| Peptidyl arginine déiminase 4 (PADI4) | ENSG00000159339 | ARNm |
| Domaine PDZ et LIM 7 (PDLIM7) | ENSG00000196923 | ARNm |
| Récepteur du facteur d'activation des plaquettes (PTAFR) | ENSG00000169403 | ARNm |
| Protéine de liaison RAN 6 (RANBP6) | ENSG00000137040 | ARNm |
| ARN, 7SL, cytoplasmique 473, pseudogène (RN7SL473P) | ENSG00000277452 | ARNm |
| RP11-429E11.3 | ENSG00000179253.3 | ARNm |
| RP11-4B16.1 | ENSG00000279433 | microARN |
| Protéine ribosomale S20 (RPS20) | ENSG00000008988 | ARNm |
| Syntaxine 10 (STX10) | ENSG00000104915 | ARNm |
| Récepteur des cellules T bêta variable 11-2 (TRBV11-2) | ENSG00000241657 | ARNm |
| Protéine du kinétochore de Zwilch (ZWILCH) | ENSG00000174442 | ARNm |
ii) un métabolite choisi dans le groupe constitué par :
- 5-méthyluridine (ribothymidine)
- fibrinopeptide A, phosphono-ser(3)
- glyco-alpha-muricholate ;
- 3-(4-hydroxyphényl)lactate
- glucoronate
- Trans-4-hydroxyproline ;
iii) phosphatidylinositol (16:0/18:1) ; ou une combinaison de ceux-ci.

3. Ensemble de biomarqueurs selon la revendication 2, comprenant :
i) le sous-ensemble de 44 ARN figurant dans le tableau suivant :
| **Nom** | **ID de l'ensemble** | **Type** |
|---|---|---|
| Membre 4 de la famille des Acyl-CoA synthétases (AASDH) | ENSG00000157426 | ARNm |
| Répétition de l'ankyrine et domaine de la mort contenant 1A (ANKDD1A) | ENSG00000166839 | ARNm |
| Domaine de répétition de l'ankyrine 49 (ANKRD49) | ENSG00000168876 | ARNm |
| Facteur d'échange de nucléotides de guanine Rho/Rac 18 (ARHGEF18) | ENSG00000104880 | ARNm |
| Domaine BRICHOS contenant 5 (BRICD5) | ENSG00000182685 | ARNm |
| Protéine d'interaction BRCA1 avec la C-terminal hélicase 1 (BRIP1) | ENSG00000136492 | ARNm |
| Domaine BRO1 et contenant le motif CAAX (BROX) | ENSG00000162819 | ARNm |
| Coactivateur-1 du récepteur AF-1 des glucocorticoïdes (BTBD6) | ENSG00000184887 | ARNm |
| Cadre de lecture ouvert 79 du chromosome 19 (C12orf79) | ENSG00000257242 | ARN long non codant |
| Cadre de lecture ouvert 38 du chromosome 19 (C19orf38) | ENSG00000214212 | ARNm |
| Protéine kinase dépendante du calcium et de la calmoduline kinase 2 (CAMKK2) | ENSG00000110931 | ARNm |
| Calpaïne 2 (CAPN2) | ENSG00000162909 | ARNm |
| Chimérine I (CHN1) | ENSG00000128656 | ARNm |
| Cvcline-M3 (CNNM3) | ENSG00000168763 | ARNm |
| Membre 1 de la sous-famille S de la famille 2 du cytochrome P450 (CYP2S1) | ENSG00000167600 | ARNm |
| Sous-unité 2 de la dvnactine (DCTN2) | ENSG00000175203 | ARNm |
| Diacylglycérol kinase delta (DGKD) | ENSG00000077044 | ARNm |
| Inhibiteur de rétroaction du récepteur ERBB 1 (ERRFI1) | ENSG00000116285 | ARNm |
| Rétrotransposon gag like 8C (FAM127A) | ENSG00000134590 | ARNm |
| Glycoprotéine, alpha-galactosyltransférase (GGTA1P) | ENSG00000204136 | ARNm |
| Glutathion-disulfure réductase (GSR) | ENSG00000104687 | ARNm |
| Groupe 18 du complexe HLA (HCG18) | ENS G00000231074 | ARNm |
| Membre C de la famille du groupe d'histones 1 H1 (HIST1H1C) | ENSG00000187837 | ARNm |
| Membre K de la famille du groupe d'histones 1 H2B (HIST1H2BK) | ENSG00000197903 | ARNm |
| Complexe majeur d'histocompatibilité, classe II, DP Alpha 1 (HLA-DPA1) | ENSG00000231389 | ARNm |
| Complexe majeur d'histocompatibilité, classe II, DP Bêta 1 (HLA-DPB1) | ENSG00000223865 | ARNm |
| Protéine de liaison du facteur de croissance analogue à l'insuline 3 (IGFBP3) | ENSG00000146674 | ARNm |
| Protéine Ras GTPase-activating-like (IQGAP2) | ENSG00000145703 | ARNm |
| Lymphotoxine bêta (LTB) | ENSG00000227507 | ARNm |
| Méthionine adénosyltransférase 2A (MAT2A) | ENSG00000168906 | ARNm |
| Protéine ribosomale mitochondriale L22 (MRPS22) | ENSG00000175110 | ARNm |
| Protéine d'assemblage du nucléosome 1 like 2 (NAP1L2) | ENSG00000186462 | ARNm |
| Peptidyl arginine déiminase 4 (PADI4) | ENSG00000159339 | ARNm |
| Domaine PDZ et LIM 7 (PDLIM7) | ENSG00000196923 | ARNm |
| Récepteur du facteur d'activation des plaquettes (PTAFR) | ENSG00000169403 | ARNm |
| Protéine de liaison RAN 6 (RANBP6) | ENSG00000137040 | ARNm |
| ARN, 7SL, cytoplasmique 473, pseudogène (RN7SL473P) | ENSG00000277452 | ARNm |
| RP11-429E11.3 | ENSG00000179253.3 | ARNm |
| RP11-4B16.1 | ENSG00000279433 | microARN |
| Protéine ribosomale S20 (RPS20) | ENSG00000008988 | ARNm |
| Syntaxine 10 (STX10) | ENSG00000104915 | ARNm |
| Récepteur des cellules T bêta variable 11-2 (TRBV11-2) | ENSG00000241657 | ARNm |
| Domaine de répétition WD 86 (WDR86) | ENSG00000187260 | ARNm |
| Protéine du kinétochore de Zwilch (ZWILCH) | ENS G00000174442 | ARNm |
ii) le sous-ensemble des 13 métabolites suivants :
- 2-aminooctanoate
- 3-(4-hydroxyphényl)lactate
- sulfate de 4-méthoxyphénol
- hypotaurine
- lysine
- glucoronate
- Trans-4-hydroxyproline
- N-acétyl1-2-aminooctanoate
- sulfate de phénol
- γ-glutamylleucine
- 5-méthyluridine (ribothymidine)
- ibrinopeptide A, phosphono-ser(3)
- glyco-alpha-muricholate ;
et iii) le sous-ensemble des lipides suivants :
- dodécanedioate (C12:1-DC)
- phosphatidylinositol (16:0/18:1) ;
pour le diagnostic du syndrome de Brugada chez un être humain.

4. Procédé in vitro pour détecter la présence ou l'occurrence de l'un des ensembles de biomarqueurs selon l'une quelconque des revendications 1 à 3 dans un échantillon biologique d'un être humain, qui comprend les étapes suivantes :
a) détecter la présence d'au moins 6 à 44 ARN du sous-ensemble i) tel que défini dans les revendications 1 et 3 par un test choisi dans le groupe comprenant la détection par microréseau d'ARNm, la PCR en temps réel, la PCR numérique en gouttelettes ou les tests basés sur l'hybridation, et la technologie de l'ADN ramifié (bDNA) (c'est-à-dire le test QuantiGene^{®}), ou une combinaison de ces tests ; et
b) détecter au moins 7 à 13 des métabolites du sous-ensemble ii) définis dans les revendications 1 et 3 et un ou deux lipides du sous-ensemble iii) définis dans les revendications 1 et 3 par un test tel qu'un test choisi dans le groupe comprenant la MS, la GC-MS, les tests fluorimétriques ou colorimétriques, la NMR, la spectroscopie avec des nanosondes, les tests d'oligonucléotides liés à l'enzyme, ou une combinaison de ces tests ;
dans lequel la détection positive de tous les biomarqueurs par rapport à un contrôle normal permet d'identifier les patients atteints du syndrome de Brugada.

5. Procédé in vitro pour mesurer la concentration d'un ensemble de biomarqueurs selon l'une quelconque des revendications 1 à 3 dans un échantillon biologique d'un être humain, comprenant les étapes suivantes :
a) déterminer quantitativement au moins 6 à 44 ARN du sous-ensemble i) tel que défini dans les revendications 1 et 3 par une technique quantitative telle que la PCR quantitative, de préférence la PCR en temps réel, la PCR numérique en gouttelettes, le test Quantigene ou une combinaison de ces techniques ; et
b) déterminer quantitativement au moins 7 à 13 des métabolites du sous-ensemble ii) défini dans les revendications 1 et 3 par une technique choisie parmi la CLHP, la SM, l'analyse RMN, l'ELONA ou une combinaison de ces techniques ; et
c) déterminer quantitativement l'un ou les deux lipides du sous-ensemble iii) défini dans les revendications 1 et 3 par une technique choisie parmi la CLHP, la SM, l'analyse RMN ou une combinaison de ces techniques ;
dans lequel une augmentation de la concentration des biomarqueurs par rapport à un contrôle normal indique un diagnostic de syndrome de Brugada.

6. Procédé in vitro selon l'une quelconque des revendications 4 à 5, dans lequel l'échantillon biologique est choisi dans le groupe constitué par le sang total, le plasma, le sérum, le sang périphérique et les PBMC.

7. Procédé in vitro selon l'une quelconque des revendications 4 à 6, dans lequel l'être humain est asymptomatique.

8. Procédé in vitro selon l'une quelconque des revendications 4 à 7, dans lequel l'être humain présente un risque élevé de syndrome de Brugada en raison d'antécédents familiaux, d'événements antérieurs de fibrillation cardiaque auriculaire et/ou ventriculaire, de diabète ou d'obésité.

9. Procédé in vitro selon l'une quelconque des revendications 4 à 8, dans lequel l'être humain est âgé d'environ 40 ans ou moins.

10. Utilisation d'un kit comprenant des oligonucléotides qui sont au moins 85, 90, 95, 96, 97, 98, 99 ou 100 % complémentaires à chacun des ARN du sous-ensemble i) selon l'une quelconque des revendications 1 à 3, dans lequel lesdits oligonucléotides sont des amorces ou des sondes éventuellement attachées à un support solide, pour le diagnostic du syndrome de Brugada chez un être humain.
